# EUROPÄISCHE PATENTSCHRIFT

(11) **EP 0 433 884 B1**
(45) Veröffentlichungstag und Bekanntmachung des Hinweises auf die Patenterteilung: **10.03.1993**
(21) Anmeldenummer: 90124062.2
(22) Anmeldetag: 13.12.1990
(51) Int. Cl.: C07C 255/29, C07C 253/30

(54) **Verfahren zur Herstellung von alpha-Formylaminonitrilen**
Process for the preparation of alpha-formylaminonitriles
Procédé pour la préparation d'alpha-formylaminonitriles

(30) Priorität: 22.12.1989 DE 3942576
(43) Veröffentlichungstag der Anmeldung: 26.06.1991
(73) Patentinhaber: BASF Aktiengesellschaft, 67063 Ludwigshafen (DE)
(72) Erfinder: Fikentscher, Rolf, Dr., W-6700 Ludwigshafen (DE); Kroener, Michael, Dr., W-6800 Mannheim 31 (DE)

(56) Entgegenhaltungen:
- EP-A- 0 205 131
- DE-A- 1 950 280
- CHEMICAL ABSTRACTS, Band 73, Nr. 7, 17. August 1970, Columbus, Ohio, USA "New synthesis of alpha(N- formylamino)nitriles, alpha- (N-formylamino)amides and of alpha- and beta-amino acids" Seite 297, Spalte 2, Zusammenfassung-Nr.34 991w

## Beschreibung

Die vorliegende Erfindung betrifft ein Verfahren zur Herstellung von α-Formylaminonitrilen der allgemeinen Formeln Ia 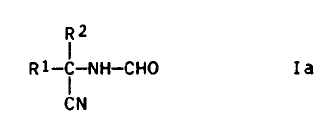
und Ib 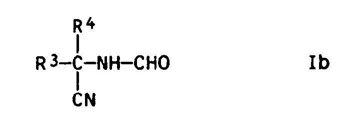
in denen die Reste R¹, R², R³ und R⁴ gleich oder verschieden sind und für Wasserstoff und/oder für unsubstituierte oder mit unter den Reaktionsbedingungen inerten Substituenten substituierte, aliphatische und/oder heteroaliphatische Reste mit 1 bis 10, cycloaliphatische und/oder heterocycloaliphatische Reste mit 3 bis 6, araliphatische Reste mit 7 bis 12, heteroaraliphatische Reste mit 4 bis 12, aromatische Reste mit 6 bis 10 und/oder heteroaromatische Reste mit 3 bis 10 Kohlenstoffatomen stehen, mit der Maßgabe, daß jeweils mindestens einer der Reste R¹ oder R² und R³ oder R⁴ ein Wasserstoffatom ist.

Gemäß der Lehre von DE-A 19 50 280 sind α-Formylaminonitrile I, in denen jeweils einer der Reste R¹ oder R² bzw. R³ oder R⁴ ungleich Wasserstoff ist, durch die säurekatalysierte Umsetzung der entsprechenden Cyanhydrine IV 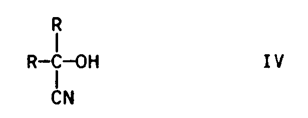
(R, R = R¹, R² oder R³, R⁴) mit Formamid III bei Temperaturen von 60 bis 180°C erhältlich. Dabei wird bevorzugt mit einem Formamid-Überschuß von 2 bis 3 mol pro Mol Cyanhydrin IV gearbeitet.

Das Verfahren der DE-A 19 50 280 liefert zwar gute Ergebnisse bei der Herstellung von α-Formylaminonitrilen, beispielsweise ist α-N-Formylalaninnitril V 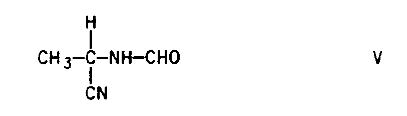
auf diese Weise in einer Ausbeute von 82 % der Theorie, ausgehend von Acetaldehydcyanhydrin, erhältlich, dennoch erschien das Verfahren in einigen Punkten als verbesserungsfähig. So entstehen z.B. bei der Herstellung von α-N-Formylalaninnitril nach dem Verfahren dieser Schrift aufgrund einer Reihe nicht näher bekannter Nebenreaktionen pro Liter Reaktionsflüssigkeit 4 bis 6 l Abgas, das im wesentlichen aus einem Kohlenmonoxid/Kohlendioxid-Gasgemisch besteht, welches noch etwa 5 Vol.-% Cyanwasserstoff enthält und daher unter aufwendigen Sicherheitsmaßnahmen sorgfältig entsorgt werden muß. Infolge dieser Gasentwicklung und damit verbundener Siedeverzüge ist die sichere Reaktionsführung erschwert, weswegen der Reaktor mit zusätzlichen, der Betriebssicherheit dienenden Vorrichtungen ausgerüstet werden muß.

Infolge der erwähnten Nebenreaktionen beträgt die Formylaminonitril-Ausbeute bei diesem Verfahren bezüglich des eingesetzten Formamids nur 75 % der Theorie. Des weiteren wird unter den angewandten Reaktionsbedingungen die Nitrilgruppe des Formylaminonitrils als auch der Cyanhydrine zum Teil durch das gebildete Reaktionswasser partiell zum Carbonsäureamid hydrolysiert, wodurch gleichfalls Ausbeuteverluste entstehen.

Der vorliegenden Erfindung lag deshalb die Aufgabe zugrunde, ein Verfahren zur Herstellung von α-Formylaminonitrilen I, also Ia und Ib, zu finden, welches frei von diesen Nachteilen, die wirtschaftliche Herstellung von I ermöglicht.

Dementsprechend wurde ein Verfahren zur Herstellung von α-Formylaminonitrilen der allgemeinen Formel Ia 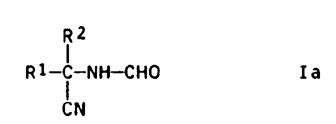
und Ib 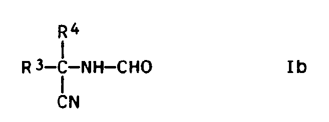
in denen die Reste R¹, R², R³ und R⁴ gleich oder verschieden sind und für Wasserstoff und/oder für unsubstituierte oder mit unter den Reaktionsbedingungen inerten Substituenten substituierte, aliphatische und/oder heteroaliphatische Reste mit 1 bis 10, cycloaliphatische und/oder heterocycloaliphatische Reste mit 3 bis 6, araliphatische Reste mit 7 bis 12, heteroaraliphatische Reste mit 4 bis 12, aromatische Reste mit 6 bis 10 und/oder heteroaromatische Reste mit 3 bis 10 Kohlenstoffatomen stehen, mit der Maßgabe, daß jeweils mindestens einer der Reste R¹ oder R² und R³ oder R⁴ ein Wasserstoffatom ist, gefunden, das dadurch gekennzeichnet ist, daß man ein Iminodiacetonitril der allgemeinen Formel II 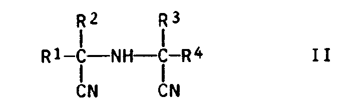
in der die Reste R¹, R², R³ und R⁴ die oben angegebene Bedeutung haben,
mit Formamid der Formel III 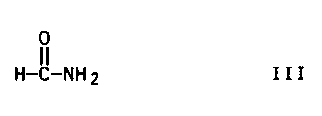
in Gegenwart von Ameisensäure oder mit einer Ameisensäure-liefernden Verbindung in Gegenwart von Säuren umsetzt.

Der Mechanismus der erfindungsgemäßen Umsetzung wurde nicht näher untersucht, es wird jedoch dabei zumindest formal gemäß Reaktionsgleichung (1) 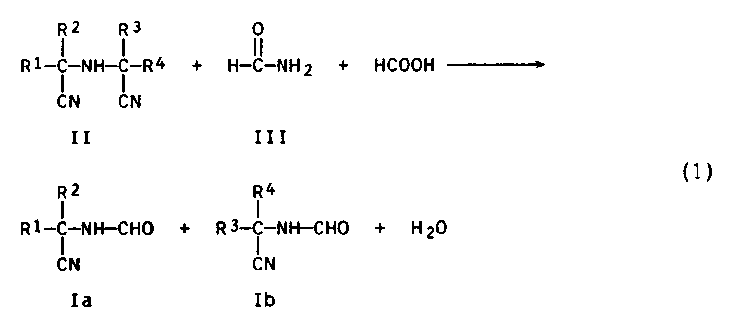
ein Molekül des Iminodiacetonitrils II mit je einem Molekül Formamid III und Ameisensäure zu den α-Formylaminonitrilen Ia und Ib umgesetzt. Dabei entsteht nach der Stöchiometrie pro Mol umgesetztem II ein Mol Reaktionswasser. Bei dieser Verfahrensweise dient die eingesetzte Ameisensäure somit sowohl als Reaktionspartner als auch als Säurekatalysator.

Die sowohl als Reaktionspartner als auch als Katalysator fungierende Ameisensäure kann in stöchiometrischer oder überschüssiger Menge bezüglich des Iminodiacetonitrils II angewandt werden. Im allgemeinen wird mit Ameisensäuremengen von 1 bis 2 mol pro Mol II gearbeitet, vorzugsweise wird jedoch ein leichter Überschuß an Ameisensäure in Höhe von 1,001 bis 2, vorteilhaft von 1,05 bis 1,5 und insbesondere von 1,1 bis 1,3 mol Ameisensäure pro Mol des Iminodiacetonitrils II eingesetzt.

Bei einer besonders vorteilhaften Ausführung des erfindungsgemäßen Verfahrens werden anstelle von Ameisensäure Mischungen aus Ameisensäure und Alkylformiaten (HCOOAlk) verwendet, wobei die einzelnen Komponenten dieser Mischungen, nämlich Ameisensäure und Alkylformiat, gemeinsam oder getrennt zum Reaktionsansatz gegeben werden können. Dabei kann das zugesetzte Alkylformiat mutmaßlich die Rolle der Ameisensäure als Reaktionspartner übernehmen, so daß im Prinzip nur noch die Anwesenheit katalytischer Mengen Ameisensäure im Reaktionsansatz erforderlich ist. Rein formal läßt sich diese erfindungsgemäße Variante der Umsetzung durch die Reaktionsgleichung (2) beschreiben:

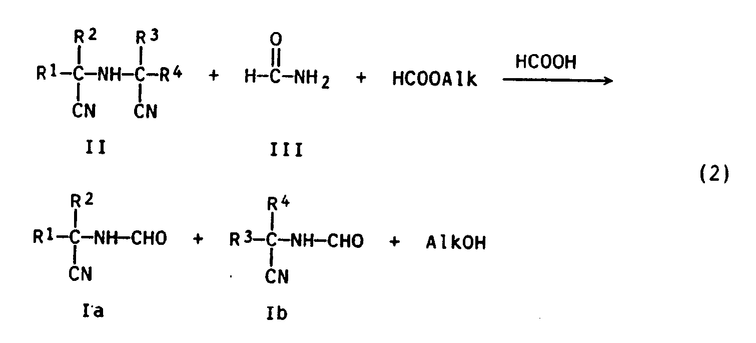

Zur Durchführung dieser Variante des erfindungsgemäßen Verfahrens können im Prinzip alle Ester der Ameisensäure benutzt werden. Aus Kostengründen sowie aufgrund ihrer leichten Verfügbarkeit werden jedoch C₁- bis C₆-, insbesondere C₁- bis C₄-Alkylformiate und speziell Methylformiat bevorzugt angewandt. Dazu können Gemische dieser Ameisensäureester oder bevorzugt die einzelnen Alkylformiate eingesetzt werden.

Bei der Verwendung von Alkylformiaten als Reaktionspartner des Iminodiacetonitrils II wird formal pro Mol II 1 mol des Alkohols AlkOH freigesetzt. Der bei der Reaktion entstehende Alkohol kann im Zuge der Aufarbeitung des Reaktionsgemisches destillativ zurückgewonnen und weiter verwendet werden. Somit entsteht bei dieser Variante formal kein Reaktionswasser. Eventuell doch in der Reaktormischung vorhandenes Wasser, sei es beispielsweise durch wasserhaltige Reaktanten in die Umsetzung eingeschleppt oder sei es durch die von den restlichen, katalytischen Mengen an Ameisensäure bewirkte Umsetzung gemäß Gleichung (1) entstanden, wirkt sich, anscheinend infolge der Anwesenheit des Alkylformiates, nicht nachteilig auf die Ausbeute der Umsetzung aus, d.h. es findet in Anwesenheit der Alkylformiate offenbar keine meßbare Partialhydrolyse der α-Formylaminonitrile I, gemäß Reaktionsgleichung (3) statt.

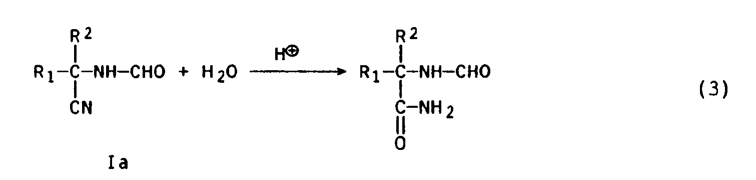

Die Alkylformiate können in praktisch jedem Mengenverhältnis bezüglich der Iminodiacetonitrile II eingesetzt werden. Verwendet man unterstöchiometrische Mengen des Alkylformiats, so ist zur Erzielung eines vollständigen Umsatzes von II ein entsprechend der Stöchiometrie der Reaktionsgleichung (1) und (2) höherer Anteil von Ameisensäure im Reaktionsansatz erforderlich. Bei der Verwendung von bezüglich II stöchiometrischen oder überschüssigen Mengen an Alkylformiat wird die Ameisensäure im allgemeinen nur in der zur Katalyse der Umsetzung erforderlichen Menge benötigt. Vorteilhaft werden die Alkylformiate in Mengen von 1 bis 2 mol pro Mol Iminodiacetonitril II eingesetzt. In diesem Falle ist im allgemeinen der Zusatz von 0,01 bis 1, vorzugsweise von 0,05 bis 0,9 und insbesondere von 0,1 bis 0,3 mol Ameisensäure pro Mol II für einen zügigen Verlauf der Reaktion ausreichend.

Bei der Verwendung von bezüglich II stöchiometrischen oder überschüssigen Mengen an Alkylformiat kann die zur Katalyse der Umsetzung benötigte Ameisensäure durch andere Säuren, vorzugsweise Mineralsäuren, ersetzt werden. Bevorzugt werden dazu unter den Reaktionsbedingungen nichtoxidierend wirkende, wasserfreie Mineralsäuren, wie Chlorwasserstoff, Fluorwasserstoff oder Tetrafluoroborsäure verwendet. Die betreffende Mineralsäure wird dabei in der Regel in Mengen von 0,01 bis 1, vorzugsweise von 0,05 bis 0,9 und insbesondere von 0,1 bis 0,3 mol Säure pro Mol II der Reaktionsmischung zugeführt.

Alle Varianten des erfindungsgemäßen Verfahrens können mit bezüglich des eingesetzten Iminodiacetonitrils II äquimolaren Mengen an Formamid durchgeführt werden, vorzugsweise werden aber Überschüsse an Formamid angewandt. In der Regel wird mit 1,1 bis 2,0 mol, vorzugsweise mit 1,2 bis 1,8 mol Formamid pro Mol II gearbeitet. Höhere Überschüsse sind möglich.

Alle Varianten des erfindungsgemäßen Verfahrens werden in der Regel bei Temperaturen von 20 bis 150 °C, vorteilhaft von 60 bis 120°C und vorzugsweise bei 80 bis 100°C, betrieben. Alle Varianten können unter Atmosphärendruck ausgeübt werden, zweckmäßigerweise wird jedoch unter erhöhtem Druck, insbesondere unter dem Eigendruck des Reaktionssystems gearbeitet. Diese Verfahrensweise ist insbesondere dann vorteilhaft, wenn niedrigsiedende Alkylformiate verwendet werden.

Das erfindungsgemäße Verfahren kann in seinen beiden Hauptvarianten, also in An- oder Abwesenheit von Alkylformiaten, sowohl diskontinuierlich in Rührkesseln, vorzugsweise in Rührautoklaven, als auch kontinuierlich in Rohrreaktoren, oder vorteilhaft in druckstabilen Rührkesselkaskaden, durchgeführt werden. Die Aufarbeitung der Reaktionsmischung und die Isolierung des oder der Reaktionsprodukte(s) können nach herkömmlichen Verfahren wie Destillation, Extraktion oder Kristallisation erfolgen. Bei destillierbaren Produkten ist die destillative Aufarbeitung des Reaktionsaustrages bevorzugt.

Das erfindungsgemäße Verfahren ist praktisch universell zur Herstellung von α-Formylaminonitrilen I aus den betreffenden Iminodiacetonitrilen II geeignet. So können die Verbindungen I, also Ia und Ib, aus den Iminodiacetonitrilen II, in denen die Reste R¹, R², R³ und R⁴ gleich oder verschieden sind und für Wasserstoff und/oder für unsubstituierte oder mit unter den Reaktionsbedingungen inerten Substituenten substituierte, aliphatische und/oder heteroaliphatische Reste mit 1 bis 10, cycloaliphatische und/oder heterocycloaliphatische Reste mit 3 bis 6, araliphatische Reste mit 7 bis 12, heteroaraliphatische Reste mit 4 bis 12, aromatische Reste mit 6 bis 10 und/oder heteroaromatische Reste mit 3 bis 10 Kohlenstoffatomen stehen, mit der Maßgabe, daß jeweils mindestens einer der Reste R¹ oder R² und R³ oder R⁴ ein Wasserstoffatom ist, vorteilhaft hergestellt werden.

Die Reste R¹, R², R³ und/oder R⁴ können mit unter den Reaktionsbedingungen inerten Substituenten, wie Halogen-, C₁- bis C₄-Alkyl-, C₁- bis C₄-Alkoxy-oder C₁- bis C₄-Halogenalkoxy-Gruppen substituiert sein. Die Anzahl der Substituenten ist im allgemeinen für den Erfolg der erfindungsgemäßen Umsetzung nicht kritisch, üblicherweise tragen jedoch die Reste R¹ bis R⁴ nicht mehr als 3 der genannten Substituenten. Die aliphatischen Reste R¹, R², R³ und/oder R⁴ können geradkettig oder verzweigt sein. Die heteroaliphatischen Reste R¹, R², R³ und/oder R⁴ enthalten als Heteroatome bevorzugt Sauerstoffatome und zwar je nach der Größe des betreffenden Restes 1 bis 5 Sauerstoffatome, vorzugsweise in Form von Ethergruppierungen. Die Heterocycloalkyl-, Heteroaralkyl- und Heteroaryl-Reste R¹, R², R³ und/oder R⁴ können im allgemeinen 1 oder 2 der Heteroatome Stickstoff, Sauerstoff und/oder Schwefel enthalten.

Um die Breite der Anwendbarkeit des erfindungsgemäßen Verfahrens zu veranschaulichen, seien im folgenden eine Reihe von Gruppen genannt, die anstelle der Reste R¹ bis R⁴, in den erfindungsgemäß aus den entsprechend substituierten Iminodiacetonitrilen II erhältlichen, α-Formylaminonitrilen Ia und Ib, stehen können: Wasserstoff, Methyl, Ethyl, Propyl, Isopropyl, Butyl, Isobutyl, Hexyl, Phenyl, Benzyl, Phenylethyl, Phenylpropyl, Phenylisopropyl, 4-Methylbenzyl, 4-Methoxybenzyl, 2,6-Dichlorphenyl, 4-Chlorbenzyl, 4-Hydroxybenzyl, 4-Chlorphenyl, Cyclopropyl, Cyclohexyl, 1-Methylcyclopropyl, 2-Pyrrolidon-3-ethylenyl, Tetrahydropyran-3-yl, Tetrahydrothiopyran-3-yl, Pyridin-2-yl, Furan-3-yl, 5-Hydroxymethyl-furan-2-yl, Pyrrol-2-yl, Imidazol-4-methylenyl, 3-Thienyl, (1H)-Indol-3-methylenyl.

Des weiteren können nach dem erfindungsgemäßen Verfahren α-N-Formylaminonitrile I, d.h. Ia und Ib, hergestellt werden, deren Reste R¹ und/oder R² bzw. R³ und/oder R⁴ Ether- oder Polyether-Gruppen sind. Beispielsweise können α-N-Formylaminonitrile mit den im folgenden beispielhaft aufgeführten Gruppen R¹, R², R³ und/oder R⁴ vorteilhaft nach dem erfindungsgemäßen Verfahren erhalten werden: Methoxy, Propoxy, Hexoxy, Methoxyethylenyl, Propoxyethylenyl, Methoxypropylenyl, Ethoxypropylenyl, Propoxypropylenyl, Hexoxypropylenyl, Oxyethylenmethylether, Oxyethylenpropylether, Bis(oxyethylen)methylether, Bis(oxyethylen)ethylether, Bis(oxyethylen)hexylether, Tetrakis(oxyethylen)methylether, Tetrakis(oxyethylen)ethylether.

Die als Ausgangsverbindungen benutzten Iminodiacetonitrile II sind beispielsweise nach dem Verfahren der DE-A 14 93 752, durch Umsetzung der entsprechenden Cyanhydrine IV (R = R¹, R² und/oder R³, R⁴) mit Ammoniak, gemäß Reaktionsgleichung (4) erhältlich: 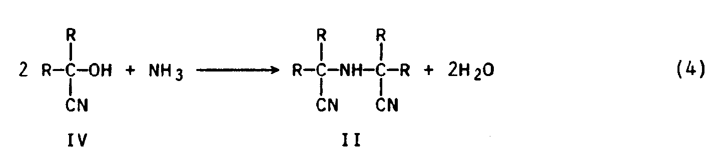
Alternativ hierzu sind die Iminodiacetonitrile II durch die Umsetzung eines Cyanhydrins IV mit einem α-Aminonitril VI, gemäß dem Verfahren der DE-A 32 42 193, entsprechend der Reaktionsgleichung (5) erhältlich (R = R¹, R² und/oder R³, R⁴):

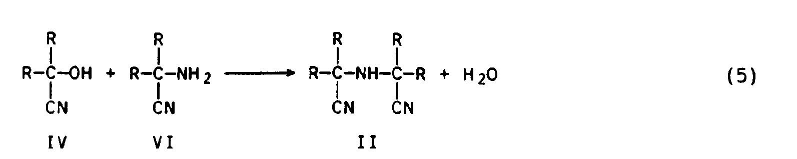

Dabei eignet sich das Verfahren der DE-A 32 42 193 besonders vorteilhaft zur Herstellung von Iminodiacetonitrilen II, in denen die Reste R¹ und R² von den Resten R³ und R⁴ verschieden sind. Da die Iminodiacetonitrile II in der Regel nicht oder nur in geringem Maße wasserlöslich sind, kann das bei ihrer Herstellung anfallende Reaktionswasser, vor ihrem Einsatz im erfindungsgemäßen Verfahren durch eine einfache Phasentrennung weitgehend abgetrennt werden.

Der Restwassergehalt der so erhaltenen Iminodiacetonitrile wirkt sich in der Regel und insbesondere bei der Verwendung von Alkylformiaten im erfindungsgemäßen Verfahren, nicht nachteilig auf das Verfahrensergebnis aus. Es kann sich bei der Verwendung restwasserhaltiger Iminodiacetonitrile II als auch bei Verwendung wasserhaltiger Ameisensäure als vorteilhaft herausstellen, die Menge des eingesetzten Alkylformiates entsprechend dem Wassergehalt des Reaktionsansatzes zu erhöhen. In der Regel werden pro Mol im Reaktionsansatz enthaltenen Wassers zusätzlich 0,5 bis 2 mol, vorzugsweise äquimolare Mengen an Alkylformiat zugesetzt.

Die dem erfindungsgemäßen Verfahren zugrundeliegende Umsetzung ist als solche unbekannt und ihr glattes Gelingen überraschend. Des weiteren lassen sich nach dem erfindungsgemäßen Verfahren überraschenderweise nicht nur die Nachteile des Verfahrens der DE-A 19 50 280 vermeiden, d.h. es entsteht kein Cyanwasserstoff-haltiges Abgas und die Partialhydrolyse der gebildeten Formylaminonitrile wird unterdrückt, wodurch letztendlich die Ausbeute an I wesentlich verbessert wird, das erfindungsgemäße Verfahren ist auch dadurch wirtschaftlicher, daß der Verbrauch an Formamid um die Hälfte gesenkt werden kann. Dies ist darauf zurückzuführen, daß die Funktion des Formamids im erfindungsgemäßen Verfahren anscheinend teilweise durch die, von den Einsatzkosten her, günstigere Ameisensäure oder vom Alkylformiat übernommen wird.

Die erfindungsgemäß erhältlichen α-Formylaminonitrile können, wie in DE-A 19 50 280 beschrieben, zu den entsprechenden Aminosäuren verseift werden. Des weiteren können aus den erfindungsgemäß erhältlichen α-Formylaminonitrilen durch thermische Cyanwasserstoff-Eliminierung gemäß den Verfahren der DE-A 16 68 038 und EP-B 184 074 die entsprechenden N-Formyl-N-Alkene erhalten werden, welche als Monomere Verwendung finden. Beispielsweise kann nach diesen Verfahren das erfindungsgemäß erhältliche α-N-Formylalaninnitril zu N-Vinylformamid pyrolysiert werden, welches gemäß EP-B 71 050 und EP-A 23 19 01 zu basischen Polymerisaten verarbeitet wird, die beispielsweise als Hilfsmittel bei der Papierveredelung verwendet werden.

### Beispiele

### Beispiel 1

### α-N-Formylalaninnitril

184,5 g Iminodipropionitril (1,5 mol; R¹, R² = R³, R⁴ = H, Methyl) wurden mit 81 g Formamid (1,8 mol), 108 g Methylformiat (1,8 mol) und 34,5 g Ameisensäure (0,75 mol) versetzt und unter Rühren in einem Autoklaven 7 h auf 90°C erwärmt. Dabei stellte sich im Autoklaven ein Druck von 2,3 bar ein. Anschließend wurde der Reaktor auf Raumtemperatur abgekühlt und entspannt. Der Reaktionsaustrag enthielt noch 8,2 g nicht umgesetztes Iminodipropionitril, entsprechend einem Umsatz von 95,6 %. Nach der destillativen Aufarbeitung des Reaktionsaustrages wurde α-N-Formylalaninnitril in einer Ausbeute von 91,6 %, bezogen auf umgesetztes Dinitril, erhalten.

### Beispiel 2

### α-N-Formylalaninnitril

Ein restwasserhaltiges Iminodipropionitril, welches außer 184,5 g Iminodipropionitril noch 18 g Wasser enthielt, wurde mit 81 g Formamid (1,8 mol), 168 g Methylformiat (2,8 mol) und 34,5 g Ameisensäure (0,75 mol) versetzt und unter Rühren in einem Autoklaven 3 h auf 100°C erhitzt. Dabei stellte sich im Autoklaven ein Druck von 4,3 bar ein. Anschließend wurde der Reaktor auf Raumtemperatur abgekühlt und entspannt. Der Reaktionsaustrag enthielt noch 4,9 g nicht umgesetztes Dinitril, entsprechend einem Umsatz von 97,3 %. Nach der destillativen Aufarbeitung des Reaktionsaustrages wurde α-N-Formylalaninnitril in einer Ausbeute von 92,5 %, bezogen auf das umgesetzte Dinitril, erhalten.

### Beispiel 3

### α-N-Formylalaninnitril

Es wurde der gleiche Reaktionsansatz wie in Beispiel 2 gewählt, die Umsetzung wurde jedoch bei 90°C über einen Zeitraum von 7 h durchgeführt. Bei dieser Reaktionsführung wurde im Autoklaven ein Eigendruck von 2,4 bar gemessen. Der Reaktionsaustrag wurde wie in Beispiel 2 beschrieben aufgearbeitet. Ausbeute: 96,3 % (bezogen auf umgesetztes Dinitril).

### Beispiel 4

### α-N-Formylalaninnitril

55,4 g Iminodipropionitril (0,45 mol) wurden mit 34,4 g Formamid (0,75 mol) und 31 g Ameisensäure (0,67 mol) unter Rühren 4 h auf 90°C erhitzt. Der Reaktionsaustrag wurde destillativ aufgearbeitet. Der Umsatz betrug 99 %. Ausbeute: 86,7 % (bezogen auf umgesetztes Dinitril).

### Beispiel 5

### α-N-Formylalaninnitril und α-N-Formylaminobuttersäurenitril

61,7 g α-Methyl-α′-ethyl-iminodiacetonitril (0,45 mol; R¹, R²: H, Methyl; R³, R⁴: H, Ethyl) wurden mit 28,4 g Formamid (0,63 mol) und 29 g Ameisensäure (0,63 mol) unter Rühren 6,5 h auf 90°C erhitzt. Der Reaktionsaustrag wurde destillativ aufgearbeitet. Der Umsatz betrug 98 %. Bezogen auf das umgesetzte Dinitril wurden α-N-Formylalaninnitril und α-N-Formylaminobuttersäurenitril in Ausbeuten von 88,2 bzw. 90,7 % der Theorie erhalten.

### Beispiel 6

### N-Formylglycinnitril und α-N-Formylleucinnitril

75, 5 g α-Isobutyl-iminodiacetonitril (0,5 mol; R¹, R²: H, Isobutyl; R³, R⁴: H, H) wurden mit 31,5 g Formamid (0,73 mol) und 32,2 g Ameisensäure (0,7 mol) unter Rühren 4 h auf 90°C erwärmt. Der Reaktionsaustrag wurde destillativ aufgearbeitet. Der Umsatz betrug 97 %. Bezogen auf umgesetztes Dinitril wurden N-Formylglycinnitril und α-N-Formylleucinnitril in Ausbeuten von 70 bzw. 82,6 % der Theorie erhalten.

### Beispiel 7

### α-N-Formylalaninnitril und α-N-Formylvalinnitril

75,5 g α-Methyl-α′-isopropyl-iminodiacetonitril (0,5 mol; R¹, R²: H, Methyl; R³, R⁴: H, Isopropyl) wurden mit 31,5 g Formamid (0,73 mol) und 32,2 g Ameisensäure (0,7 mol) unter Rühren 6 h auf 90°C erhitzt. Der Reaktionsaustrag wurde destillativ aufgearbeitet. Der Umsatz betrug 96 % Bezogen auf umgesetztes Dinitril wurden α-N-Formylalaninnitril und α-N-Formylvalinnitril in Ausbeuten von 80 bzw. 90 % der Theorie erhalten.

## Patentansprüche

1. Verfahren zur Herstellung von α-N-Formylaminonitrilen der allgemeinen Formel Ia 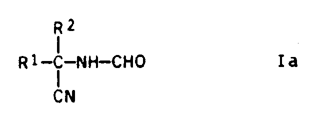 und Ib 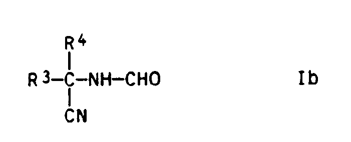 in denen die Reste R¹, R², R³ und R⁴ gleich oder verschieden sind und für Wasserstoff und/oder für unsubstituierte oder mit unter den Reaktionsbedingungen inerten Substituenten substituierte, aliphatische und/oder heteroaliphatische Reste mit 1 bis 10, cycloaliphatische und/oder heterocycloaliphatische Reste mit 3 bis 6, araliphatische Reste mit 7 bis 12, heteroaraliphatische Reste mit 4 bis 12, aromatische Reste mit 6 bis 10 und/oder heteroaromatische Reste mit 3 bis 10 Kohlenstoffatomen stehen, mit der Maßgabe, daß jeweils mindestens einer der Reste R¹ oder R² und R³ oder R⁴ ein Wasserstoffatom ist, dadurch gekennzeichnet, daß man ein Iminodiacetonitril der allgemeinen Formel II 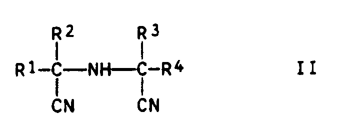 in der die Reste R¹, R², R³ und R⁴ die oben angegebene Bedeutung haben,
mit Formamid der Formel III 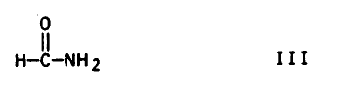 in Gegenwart von Ameisensäure oder mit einer Ameisensäure-liefernden Verbindung in Gegenwart von Säuren umsetzt.

2. Verfahren nach Anspruch 1, dadurch gekennzeichnet, daß man die Umsetzung in Gegenwart eines C₁- bis C₆-Alkylformiates als Ameisensäure-liefernder Verbindung und Ameisensäure durchführt.

3. Verfahren nach Anspruch 1, dadurch gekennzeichnet, daß man die Umsetzung in Gegenwart eines C₁- bis C₆-Alkylformiates und einer Mineralsäure durchführt.

4. Verfahren nach Anspruch 1, dadurch gekennzeichnet, daß man die Umsetzung bei Temperaturen von 20 bis 150°C ausführt.

5. Verfahren nach Anspruch 1, dadurch gekennzeichnet, daß man die Umsetzung unter dem Eigendruck des Reaktionssystems durchführt.

6. Verfahren nach Anspruch 1, dadurch gekennzeichnet, daß man Iminodiacetonitrile II umsetzt, in denen die Reste R¹, R², R³ und R⁴ gleich oder verschieden sind und für Wasserstoff, C₁- bis C₁₀-Alkyl-, C₃- bis C₆-Cycloalkyl-, C₇- bis C₁₁-Aralkyl- und/oder C₄- bis C₁₁-Heteroaralkylgruppen und/oder für sauerstoffhaltige C₁- bis C₁₀-Heteroalkylgruppen mit 1 bis 5 Sauerstoffatomen stehen und wobei jeweils mindestens einer der Reste R¹ oder R² und R³ oder R⁴ ein Wasserstoffatom ist.

7. Verfahren nach Anspruch 1, dadurch gekennzeichnet, daß man Iminodiacetonitrile II umsetzt, in denen die Reste R¹, R², R³ und R⁴ gleich oder verschieden sind und für Wasserstoff und C₁- bis C₁₀-Alkylgruppen stehen, wobei jeweils mindestens einer der Reste R¹ oder R² und R³ oder R⁴ ein Wasserstoffatom ist.

## Claims

1. A process for preparing α-N-formylamino nitriles of the formula Ia 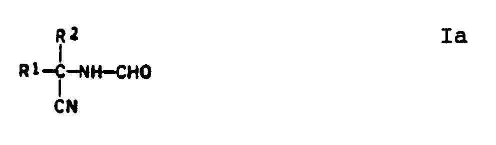 and Ib 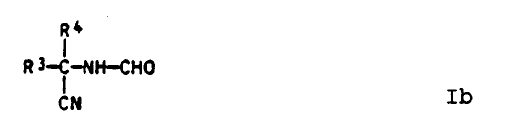 where R¹, R², R³ and R⁴ are identical or different and each is hydrogen or unsubstituted or substituted, with substituents which are inert under the reaction conditions, aliphatic or heteroaliphatic radicals with 1 to 10, cycloaliphatic or heterocycloaliphatic radicals with 3 to 6, araliphatic radicals with 7 to 12, heteroaraliphatic radicals with 4 to 12, aromatic radicals with 6 to 10 or heteroaromatic radicals with 3 to 10 carbon atoms, with the proviso that at least one of R¹ and R² or R³ and R⁴ is hydrogen, which comprises reacting an iminodiacetonitrile of the formula II 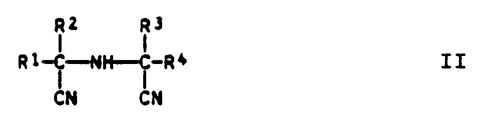 where R¹, R², R³ and R⁴ have the abovementioned meanings, with formamide of the formula III 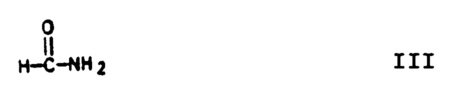 in the presence of formic acid or with a compound which provides formic acid, in the presence of acids.

2. A process as claimed in claim 1, wherein the reaction is carried out in the presence of a C₁-C₆-alkyl formate as compound providing formic acid, and of formic acid.

3. A process as claimed in claim 1, wherein the reaction is carried out in the presence of a C₁-C₆-alkyl formate and of a mineral acid.

4. A process as claimed in claim 1, wherein the reaction is carried out at from 20 to 150°C.

5. A process as claimed in claim 1, wherein the reaction is carried out under the autogenous pressure of the reaction system.

6. A process as claimed in claim 1, wherein an iminodiacetonitrile II where R¹, R², R³ and R⁴ are identical or different and are each hydrogen, C₁-C₁₀-alkyl, C₃-C₆-cycloalkyl, C₇-C₁₁-aralkyl or C₄-C₁₁-heteroaralkyl, or C₁-C₁₀-heteroalkyl with 1 to 5 oxygen atoms, and where not less than one of R¹ or R² and R³ or R⁴ is hydrogen, is reacted.

7. A process as claimed in claim 1, wherein an iminodiacetonitrile II where R¹, R², R³ and R⁴ are identical or different and are each hydrogen or C₁-C₁₀-alkyl, and where not less than one of R¹ or R² and R³ or R⁴ is hydrogen, is reacted.

## Revendications

1. Procédé de préparation de α-N-formylaminonitrile de formules générales Ia 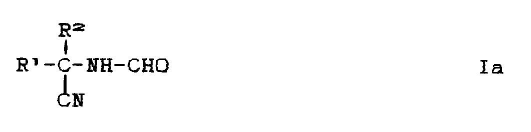 et Ib 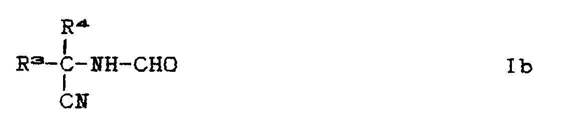 dans lesquelles les restes R¹, R², R³ et R⁴ sont identiques ou différents et sont mis pour des atomes d'hydrogène et/ou pour des restes non substitués ou substitués par des substituants inertes dans les conditions de la réaction, aliphatiques et/ou hétéroaliphatiques ayant 1 à 10 atomes de carbone, des restes cycloaliphatiques et/ou hétérocycloaliphatiques ayant 3 à 6 atomes de carbone, des restes araliphatiques ayant 7 à 12 atomes de carbone, des restes hétéroaraliphatiques ayant 4 à 12 atomes de carbone, des restes aromatiques ayant 6 à 10 atomes de carbone et/ou des restes hétéroaromatiques ayant 3 à 10 atomes de carbone, à condition qu'à chaque fois l'un des restes R¹ ou R² et R³ ou R⁴ est un atome d'hydrogène, caractérisé en ce que l'on fait réagir un iminodiacétonitrile de formule générale II 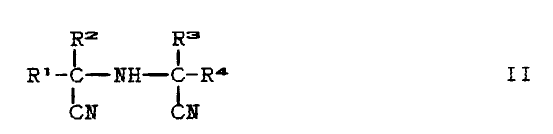 dans laquelle les restes R¹, R², R³ et R⁴ ont la signification indiquée ci-dessus, avec un formamide de formule III 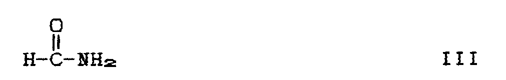 en présence d'acide formique ou avec un composé produisant de l'acide formique en présence d'acides.

2. Procédé selon la revendication 1, caractérisé en ce que l'on effectue la réaction en présence d'un formiate d'alkyle en C₁-C₆ en tant que composé produisant de l'acide formique, et en présence d'acide formique.

3. Procédé selon la revendication 1, caractérisé en ce que l'on effectue la réaction en présence d'un formiate d'alkyle en C₁-C₆ et d'un acide minéral.

4. Procédé selon la revendication 1, caractérisé en ce que l'on effectue la réaction à des températures de 20 à 150°C.

5. Procédé selon la revendication 1, caractérisé en ce que l'on effectue la réaction sous la pression propre du système réactionnel.

6. Procédé selon la revendication 1, caractérisé en ce que l'on fait réagir des iminodiacétonitriles II, dans lesquels les restes R¹, R², R³ et R⁴ sont identiques ou différents et sont mis pour des atomes d'hydrogène, des groupes allyle en C₁-C₁₀, cycloalkyle en C₃-C₆, aralkyle en C₇-C₁₁ et/ou hétéroaralkyle en C₄-C₁₁ et/ou hétéroalkyle en C₁-C₁₀ contenant de l'oxygène ayant 1 à 5 atomes d'oxygène et dans lesquels à chaque fois au moins l'un des restes R¹ ou R² et R³ ou R⁴ est un atome d'hydrogène.

7. Procédé selon la revendication 1, caractérisé en ce que l'on fait réagir des iminodiacétonitriles II, dans lesquels les restes R¹, R², R³ et R⁴ sont identiques ou différents et sont mis pour des atomes d'hydrogène et des groupes allyle en C₁-C₁₀, et dans lesquels à chaque fois au moins l'un des restes R¹ ou R² et R³ ou R⁴ est un atome d'hydrogène.
